# EUROPÄISCHE PATENTSCHRIFT

(11) **EP 2 953 585 B1**
(45) Veröffentlichungstag und Bekanntmachung des Hinweises auf die Patenterteilung: **21.06.2017**
(21) Anmeldenummer: 14712571.0
(22) Anmeldetag: 07.02.2014
(51) Int. Cl.: A61F 2/36, A61F 2/44, A61B 17/72, A61F 2/30

(54) **ZWISCHENKÖRPER FÜR EIN KNOCHENIMPLANTAT UND IMPLANTATANORDNUNG**
INTERMEDIATE BODY FOR A BONE IMPLANT AND IMPLANT DEVICE
CORPS INTERMÉDIAIRE POUR IMPLANT OSSEUX ET ENSEMBLE IMPLANT

(30) Priorität: 11.02.2013 DE 102013101325
(43) Veröffentlichungstag der Anmeldung: 16.12.2015
(73) Patentinhaber: Aristotech Industries GmbH, 14943 Luckenwalde (DE)
(72) Erfinder: ANAPLIOTIS, Emmanuel, 14195 Berlin (DE); LOB, Guenter, 81377 München (DE)
(74) Vertreter: Bittner, Thomas L.
(86) Internationale Anmeldenummer: PCT/DE2014/100044
(87) Internationale Veröffentlichungsnummer: WO 2014/121789

(56) Entgegenhaltungen:
- DE-A1- 10 308 141
- US-A1- 2003 204 269
- US-A1- 2006 129 247
- US-A1- 2013 006 358
- US-B1- 8 287 598

## Beschreibung

Die Erfindung betrifft einen Zwischenkörper für ein Implantat zum Ersatz eines knöchernen Bereiches sowie eine Implantatanordnung.

### Hintergrund

Aus dem Dokument EP 0 920 289 B1 ist eine Endoprothese bekannt, bei der ein Zwischenkörper mit einem zylindrisch ausgebildeten Basiskörper vorgesehen ist, in welchem sich in axialer Richtung eine Aufnahme durch den Basiskörper hindurch erstreckt, derart, dass über stirnseitige Eintrittsöffnungen beidseitig ein Endoprothesenteil in die Aufnahme einführbar ist. Der Basiskörper ist mit zwei Halbschalen gebildet, die im montierten Zustand entlang einer Mittelebene parallel zueinander liegen und mittels Spannmitteln in Form von Schrauben miteinander verbunden sind. Mit Hilfe der Schrauben wird dann auch der endseitig eingesteckte Endabschnitt des Endoprothesenteils geklemmt und hierdurch in seiner Relativlage zum Basiskörper fixiert.

Ein vergleichbarer Zwischenkörper für eine Endoprothese ist aus dem Dokument US 2011 / 0196503 A1 bekannt. Der bekannte Zwischenkörper kann mit unterschiedlicher axialer Baulänge hergestellt sein. Zur Ausbildung unterschiedlicher Baulängen des Endabschnittes von Endoprothesenteilen verbindenden Zwischenkörpers kann ein Zwischenverbinder vorgesehen sein.

Im Dokument WO 2010 / 025704 A1 ist ein Kniearthrodese-Implantat offenbart, bei dem ein Prothesenteile verbindender Zwischenkörper mit zwei zueinander abgewinkelten Abschnitten gebildet ist.

In dem Dokument US 2003/0204269 A1 ist ein modulares Prothesensystem offenbart, bei dem mit Hilfe eines Zwischenstücks Implantatabschnitte verbunden werden. Ein Abschnitt eines ersten Bauteils wird beim Zusammenführen über einen Abschnitt eines zweiten Bauteils gestülpt, wodurch eine Klemmverbindung ausgebildet.

In dem Dokument US 2006/0129247 A1 ist eine Implantanordnung offenbart, bei der Implantate über ein Zwischenstück miteinander verbunden werden. In ähnlicher Weise offenbart das Dokument US 2013/0006358 A1 eine Implantanordnung, bei der Wirbelimplantate mittels Schraubverbindungen über ein Zwischenstück miteinander verbunden sind. Zum Feststellen der Relativlage von Implantaten und Zwischenstück ist eine Feststellschraube vorgesehen.

Im Dokument DE 103 08 141 A1 ist eine Verlängerungshülse für eine modular aufgebaute Gelenkprothese beschrieben. Um bei einer Verlängerungshülse für eine modular aufgebaute, in einen Röhrenknochen einsetzbare Gelenkprothese mit einem Steckzapfen zur Verbindung mit einem ersten benachbarten Modul der Gelenkprothese und mit einem sich an den Steckzapfen anschließenden hülsenförmigen Aufnahmeteil mit einem Aufnahmekanal zur Aufnahme eines Steckzapfens eines zweiten, dem ersten gegenüberliegenden Moduls der Gelenkprothese, deren Außenkontur zur Ausfüllung des Markraumquerschnitts des die Gelenkprothese aufnehmenden Röhrenknochens ausgebildet ist, den Mikroabrieb im Verbindungsbereich zu reduzieren, wird vorgeschlagen, das die Biegesteifigkeit der Wand der Verlängerungshülse gegenüber einer massiven, aus dem Material der Verlängerungshülse bestehenden Wand herabgesetzt ist.

Im Dokument US 8,287,598 B1 ist ein modulares Prothesensystem offenbart, bei dem zwei Endplatte mit einem zylindrischen, flexiblen Bauteil aus einem faserverstärkten Material verbunden sind. Die Befestigung der Endplatten an dem zylindrischen Bauteil erfolgt mittels eines jeweiligen Klemmrings.

### Zusammenfassung

Aufgabe der Erfindung ist es, einen Zwischenkörper für ein Implantat zum Ersatz eines knöchernen Bereiches sowie eine Implantatanordnung anzugeben, bei denen Zwischenkörper und Implantatteil(e) oder -abschnitt(e) mit verbesserter mechanischer Stabilität verbindbar sind, insbesondere auch im Fall eines nicht zylindrisch ausgebildeten Endabschnitts des Implantatteils oder -bauteils, zum Beispiel eines Endoprothesenteils.

Zur Lösung der Aufgabe sind ein Zwischenkörper für ein Implantat zum Ersatz eines knöchernen Bereiches im diaphysären und / oder metaphysären Bereich eines Knochens nach dem unabhängigen Anspruch 1 sowie eine Implantatanordnung nach dem unabhängigen Anspruch 13 geschaffen. Vorteilhafte Ausgestaltungen sind Gegenstand von abhängigen Unteransprüchen.

Im Unterschied zum Stand der Technik erfolgt das Fixieren des Endabschnittes des intramedullären Implantatteils oder -bauteils in der am Basiskörper gebildeten Aufnahme also gar nicht oder nicht ausschließlich mit Hilfe der Wandung des Basiskörpers selbst, sondern exklusiv oder ergänzend mittels der Klemmeinrichtung, welche wenigstens ein Klemmbauteil aufweist, das den in die Aufnahme am Basiskörper zuvor eingeführten Endabschnitt des Implantatteils dann in der Aufnahme fixiert. Hierbei kann vorgesehen sein, dass der eingeführte Endabschnitt zuvor bereits vorgeklemmt und dann mittels des Klemmbauteils endgültig fixiert wird. Alternativ kann vorgesehen sein, dass die Fixierung des Endabschnitts in der Aufnahme ausschließlich mit Hilfe der Klemmeinrichtung erfolgt, insbesondere mit dem verlagerbaren Klemmbauteil.

Die Klemmeinrichtung weist mehrere Klemmbauteile auf, welche an der die Aufnahme umgreifenden Wandung des Basiskörpers angeordnet sind und welche unabhängig voneinander oder wenigstens zum Teil gemeinsam zum Klemmen des zuvor in die Aufnahme eingeführten Endabschnittes relativ zur Wandung des Basiskörpers, die dann die Aufnahme und den eingeführten Endabschnitt umgreift, zur Aufnahme hin und hierbei den Endabschnitt in der Aufnahme klemmend verlagerbar sind. Die mehreren Klemmbauteile können lösbar oder nicht lösbar im Bereich der die Aufnahme umgreifenden Wandung des Basiskörpers angeordnet sein. Die mehreren Klemmbauteile können gleich oder unterschiedlich ausgeführt sein, insbesondere hinsichtlich ihrer äußeren Formgestaltung.

Die mehreren Klemmbauteile bilden eine sich in axialer Richtung des Basiskörpers erstreckende Anordnung von Klemmelementen, die wahlweise benachbart zueinander angeordnet sind. Die mehreren Klemmbauteile können in Längsrichtung des Basiskörpers nebeneinander angeordnet sein, sei es in regelmäßigen oder unregelmäßigen Abständen. Hierbei kann jedes der Klemmbauteile in einer zugeordneten Klemmbauteilaufnahme angeordnet sein. Es können auch mehrere Klemmbauteile in einer gemeinsamen Klemmbauteilaufnahme gelagert sein.

Der Endabschnitt des intramedullären Implantatteils kann verschiedenste Formen aufweisen, insbesondere hinsichtlich des Querschnitts, zum Beispiel rund oder eckig.

Die im Basiskörper gebildete Aufnahme kann als sich in axialer Richtung des Basiskörpers erstreckender Durchbruch ausgeführt sein. Alternativ verfügt der Basiskörper über zwei jeweils von den gegenüberliegenden Stirnseiten ausgehenden Aufnahmetaschen, die innerhalb des Basiskörpers mittels eines Trennabschnittes voneinander separiert sind, beispielsweise eine sich quer zur Axialrichtung des Basiskörpers erstreckende Trennwand.

In den unterschiedlichen Ausführungsformen kann die Aufnahme zylindrisch ausgeführt sein. Auch ein sich verjüngender Querschnitt der Aufnahme kann vorgesehen sein. Auf diese Weise ist beispielsweise eine konisch verlaufende Aufnahmetasche ausbildbar.

Das von der Klemmeinrichtung umfasste Klemmbauteil kann beim Verlagern in die Klemmstellung, also zur Aufnahme hin und hierbei dem Endabschnitt in der Aufnahme klemmend, in die Aufnahme selbst hinein verlagert werden, zumindest abschnittsweise, zum Beispiel mit einem Frontabschnitt, welcher der Aufnahme zugewandt ist. Hierdurch wird dann zumindest lokal der Querschnitt der Aufnahme vermindert, indem das Klemmbauteil in diese hineinverlagert wird.

Die Klemmeinrichtung kann als stufenlos einstellbare Klemmeinrichtung ausgeführt sein, bei der die Klemmbauteile stufenlos verstellbar sind beim Bewegen in die und aus der Klemmstellung heraus.

Die Verlagerung des Klemmbauteils erfolgt vorzugsweise zumindest quer zur axialen Richtung des Basiskörpers. Hierdurch kann das Klemmbauteil beispielsweise von der Seite in die in axialer Richtung verlaufende Aufnahme hinein verlagert werden, um so den eingesteckten Endabschnitt in der Aufnahme zu klemmen und zu fixieren, beispielsweise mittels Drücken gegen einen gegenüberliegenden Abschnitt der die Aufnahme umgebenden Wandung.

Zum zwangsweisen Verlagern des Klemmbauteils in die und aus der Klemmstellung kann beispielsweise ein in den Basiskörper einschraubbares Schraubelement vorgesehen sein, welches in eine zugeordnete Gewindebohrung in der Wandung eingeschraubt ist.

In einer Ausgestaltung können ein oder mehrere Klemmbauteile der Klemmeinrichtung selbst als Schraubelemente gebildet sein, die in der die Aufnahme umgebenden Wandung in zugeordneten Bohrungen mit Gewinde aufgenommen sind und mittels Drehen zur Aufnahme hin eingeschraubt oder von dieser weg bewegt werden können. Hierbei kann vorgesehen sein, dass das oder die Schraubelemente mit ihrem bezüglich der Aufnahme proximalen Ende (Fuß), welches zur Aufnahme hin und von dieser weg bewegbar ist, gegen ein Schraubklemmelement drücken, welches zumindest in der Klemmstellung gegen den in der Aufnahme angeordneten Endabschnitt des intramedullären Implantatteils drückt, so dass das proximale Ende des Schraubelementes vermittelt durch das Schraubklemmelement gegen den eingesteckten Endabschnitt drückt. Mithilfe des Klemmelementes kann der Fuß des Schraubenelementes verbreitert werden, um so eine größere Klemmfläche bereitzustellen. Das Schraubklemmelement kann kippbar am proximalen Ende angeordnet sein, sei es lösbar oder nicht lösbar in Bezug auf das Schraubelement, zum Beispiel unter Verwendung einer Kugelkopflagerung. Das Schraubklemmelement kann auf einer der Aufnahme zugewandten Seite mit einer gekrümmten Oberfläche versehen sein.

Die die Aufnahme umgreifende Wandung des Basiskörpers kann einteilig ausgeführt sein. Der Basiskörper kann als Ganzes oder auch nur im Bereich der die Aufnahme umgreifenden Wandung mehrteilig ausgeführt sein, wobei diese Mehrteiligkeit ohne die Elemente der Klemmeinrichtung gebildet ist. Der Basiskörper kann mit zwei Halbschalen gebildet sein, die mit Hilfe von Verbindungsmitteln, beispielsweise einer oder mehreren Schrauben, miteinander verbunden sind. Bei einer Ausführung kann der Basiskörper in einem Bereich seiner axialen Erstreckung, zum Beispiel hälftig, mit Hilfe von zwei Halbschalen gebildet sein, wohingegen der Basiskörper in einem anderen Bereich seiner axialen Erstreckung umlaufend einteilig ausgeführt, beispielsweise derart, dass die Aufnahme hier von einer einteiligen, umlaufenden Wandung umgriffen wird. Insbesondere bei unterschiedlicher Ausbildung der beiden Hälften des Basiskörpers kann im Inneren eine Trennwand zum Separieren zweier endseitiger Aufnahmetaschen vorgesehen sein.

Bei einer Ausgestaltung kann vorgesehen sein, dass das Klemmbauteil zumindest in einer Klemmstellung wenigstens abschnittsweise in einer zugeordneten Klemmbauteilaufnahme im Bereich der umgreifenden Wandung des Basiskörpers angeordnet ist. Die Klemmbauteilaufnahme kann beispielsweise mit einer Spalt- oder Schlitzführung für das hierin aufzunehmende Klemmbauteil gebildet sein. Das Klemmbauteil kann formschlüssig in der Klemmbauteilaufnahme angeordnet sein, zumindest in der Klemmstellung. In einer Ausführung weist die Klemmbauteilaufnahme eine quer zur Axialrichtung des Basiskörpers ausgebildete Vertiefung auf, welche sich von der äußeren Oberfläche des Basiskörpers aus in diesen hinein erstreckt. Hierin kann das Klemmbauteil zwischen einer Klemmstellung und einer gelösten Stellung verlagert werden, wobei das Klemmbauteil in der gelösten Stellung den Endabschnitt des intramedullären Implantatteils freigibt. Es können in einem oder beiden endseitigen Abschnitten des Zwischenkörpers mehrere gleich oder verschieden ausgeführte Klemmbauteilaufnahmen vorgesehen sein. Die Klemmbauteilaufnahmen können sich zum Beispiel hinsichtlich ihrer jeweiligen Baubreite unterscheiden. Die Baubreite der Klemmbauteilaufnahmen kann für die auf einer Seite des Zwischenkörpers gebildeten Klemmbauteilaufnahmen gleich oder verschieden sein. Ein mittlerer Abschnitt des Zwischenkörpers kann frei von Klemmbauteilaufnahmen sein.

Das Klemmbauteil kann lösbar an der umgreifenden Wandung aufgenommen sein. Alternativ ist das Klemmbauteil, bei Erhalt seiner relativen Verlagerbarkeit in Bezug auf die Wandung des Basiskörpers, hieran nicht lösbar angebracht.

Eine Fortbildung sieht vor, dass das Klemmbauteil zum Klemmen des zuvor in die Aufnahme eingeführten Endabschnitts in die Aufnahme hinein verlagerbar ist. Bei dieser Ausführungsform ist ein der Aufnahme zugewandte Abschnitt des Klemmbauteils zumindest in der Klemmstellung in der im Basiskörper gebildeten Aufnahme angeordnet und klemmt dort den in die Aufnahme eingeführten Endabschnitt.

Das eine oder die mehreren Klemmbauteile können zum Fixieren des Endabschnitts des Implantatteils oder -bauteils mit einem oder mehreren Gegenklemmbauteilen zusammenwirken, die im Bereich der Aufnahme gegenüberliegend angeordnet sein können. Hierbei kann vorgesehen sein, dass mit Hilfe von Spannmitteln, beispielsweise einer oder mehrerer Schrauben, Klemmbauteil und Gegenklemmbauteil beim Verlagern in die Klemmstellung gemeinsam aufeinander zu bewegt werden, so dass hierdurch der Endabschnitt des Implantatteils in der Aufnahme fixiert wird. Alternativ kann der Endabschnitt des Implantatteils, zumindest lokal, mit Hilfe des Klemmbauteils auch gegen einen dem Klemmbauteil gegenüberliegenden Wandungsabschnitt des Basiskörpers gedrückt werden.

Eine Weiterbildung kann vorsehen, dass für die mehrere Klemmbauteile individuelle und voneinander verschiedene Klemmweiten in der Aufnahme einstellbar sind. Auf diese Weise können für die mehreren Klemmbauteile lokal unterschiedliche Klemmweiten oder Klemmquerschnitte in der Aufnahme eingestellt werden. Beispielsweise kann sich die Klemmweite oder der Klemmquerschnitt in der Aufnahme von der Stirnseite zur Mitte hin verjüngen. Dieses ist zum Beispiel vorteilhaft, wenn ein konisch verlaufender Endabschnitt einer Hüftgelenk-Endoprothese im Zwischenkörper aufgenommen und fixiert werden soll. Aber nicht nur konische, sondern auch beliebige nicht zylindrisch ausgebildete Endabschnitte von Implantatteilen, zum Beispiel einem Schaft oder einem Nagel, können auf diese Weise vorteilhaft in dem Zwischenkörper für die Endoprothese fixiert werden. Die mehreren Klemmbauteile können bei dieser oder anderen Ausführungen von gleicher oder unterschiedlicher Form sein. Mit Hilfe unterschiedlicher Formgestaltung kann der vom jeweiligen Klemmbauteil in der Aufnahme bereitgestellte Klemmquerschnitt angepasst werden.

Die sich in axialer Richtung des Basiskörpers erstreckende Anordnung von Klemmelementen kann über die gesamte Länge des Basiskörpers gebildet sein.

Bei einer zweckmäßigen Ausgestaltung kann vorgesehen sein, dass das eine oder die mehrere Klemmbauteile auf einer der Aufnahme zugewandten Seite eine oberflächenseitige Klemmstruktur aufweisen. Die oberflächenseitige Klemmstruktur kann Klemmbacken, Klemmvorsprünge und / oder -vertiefungen aufweisen. Die oberflächenseitigen Klemmstrukturen sind an dem Klemmbauteil vorzugsweise frontseitig angeordnet, derart, dass die oberflächenseitige Klemmstruktur in der Klemmstellung gegen eine gegenüberliegende Oberfläche des geklemmten Endabschnitts des Implantatteils drückt. Die oberflächenseitige Klemmstruktur kann verschieden sein für die mehreren Klemmbauteile. Beispielsweise kann die oberflächenseitige Klemmstruktur eine Vertiefung aufweisen, deren Querschnitt für ein zur Stirnseite des Basiskörpers proximales Klemmbauteil größer als für ein im Vergleich hierzu zur Stirnseite distales Klemmbauteil ist. Auf diese Weise kann in der Aufnahme entlang der Klemmbauteile eine im Querschnitt ab- oder zunehmende Rinne gebildet werden, an welche sich dann der Endabschnitt des intramedullären Implantatteils beim Klemmen anlegt.

Eine vorteilhafte Ausführungsform sieht vor, dass das auf einer Innenseite der die Aufnahme umgreifenden Wandung eine rutschhemmende Oberflächenprofilierung gebildet ist. Die rutschhemmende Oberflächenprofilierung kann mit Profilierungen auf der Oberfläche des Endabschnitts des Implantatteils im Eingriff stehen.

Bevorzugt sieht eine Fortbildung vor, dass die Klemmeinrichtung mit Spannelementen gebildet ist, mit denen quer zueinander ausgerichtete Klemmkräfte ausbildbar sind, beispielsweise in unterschiedlichen Bereichen des Basiskörpers. Die Klemmkräfte können zum Beispiel im Winkel von etwa 90° zueinander ausgerichtet sein. Es kann vorgesehen sein, dass die quer zueinander ausgerichteten Klemmkräfte mit Hilfe von quer zueinander verlaufenden Schraubelementen bereitgestellt sind. So kann die Schraubrichtung in einer Hälfte des Basiskörpers quer zur Schraubrichtung in der anderen Hälfte des Basiskörpers sein.

Bei einer Ausgestaltung kann vorgesehen sein, dass das eine oder die mehreren Klemmbauteile mehrteilig ausgeführt sind. Eine mehrteilige Ausführung der Klemmbauteile ist beispielsweise dann gegeben, wenn dem Klemmbauteil ein Gegenbauteil zugeordnet ist, welches dem Klemmbauteil gegenüberliegend im Bereich der die Aufnahme umgebenden Wandung des Basiskörpers angeordnet ist. Solch einander zugeordnete Klemmbauteile können über zur Mittelachse gespiegelte Klemmstrukturen oberflächenseitig verfügen. Ein oder mehrere gemeinsame Spannmittel, zum Beispiel ein oder mehrere Schrauben, können vorgesehen sein, um Klemmbauteil und Gegenklemmbauteil zum Verlagern in die Klemmstellung aufeinander zu bewegen.

Eine Fortbildung kann vorsehen, dass das eine oder die mehreren Klemmbauteile jeweils mit einem in die die Aufnahme umgreifende Wandung des Basiskörpers eingeschraubten Schraubelement gebildet sind. Beispielsweise kann jeweils eine Schraube in einer zugeordneten Gewindebohrung aufgenommen sein. Hierdurch kann in einer Ausführung in beiden Endbereichen des Basiskörpers eine einstellbare und insoweit individuell gestaltbare Aufnahme zum Befestigen von Prothesenteilen mit beliebiger Schaftgeometrie bereitgestellt werden.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine perspektivische Darstellung einer Implantatanordnung, insbesondere Endoprothesenanordnung, mit einem Zwischenkörper, in welchem Endabschnitte von intramedullären Implantatteilen fixiert sind,
- Fig. 2: eine Darstellung der Implantatanordnung aus Fig. 1 von vorn,
- Fig. 3: eine perspektivische Darstellung der Implantatanordnung aus Fig. 1 im nicht montierten Zustand,
- Fig. 4: einen Abschnitt der Implantatanordnung aus Fig. 1 mit dem Zwischenkörper,
- Fig. 5: eine schematische Schnittdarstellung des Abschnittes aus Fig. 4 und
- Fig. 6: eine schematische Darstellung eine weiteren Zwischenkörpers für eine Implantatanordnung,
- Fig.7: eine perspektivische Darstellung einer weiteren Implantatanordnung mit einem Zwischenkörper, in welchem Endabschnitte von intramedullären Implantatteilen fixiert sind,
- Fig. 8: eine perspektivische Darstellung der Anordnung aus Fig. 7, wobei die zu verbindenden intramedullären Implantatteile getrennt vom Zwischenkörper angeordnet sind,
- Fig. 9: eine Darstellung der weiteren Anordnung aus Fig. 7, wobei die Endabschnitte der intramedullären Implantatteile geklemmt sind,
- Fig. 10: eine Schnittdarstellung der weiteren Anordnung aus Fig. 7,
- Fig. 11: eine perspektivische Schnittdarstellung eines Zwischenkörpers und
- Fig. 12: eine schematische Darstellung einer Implantatanordnung, bei der einander gegenüberliegende Aufnahmen für Implantatteile miteinander verbunden sind.

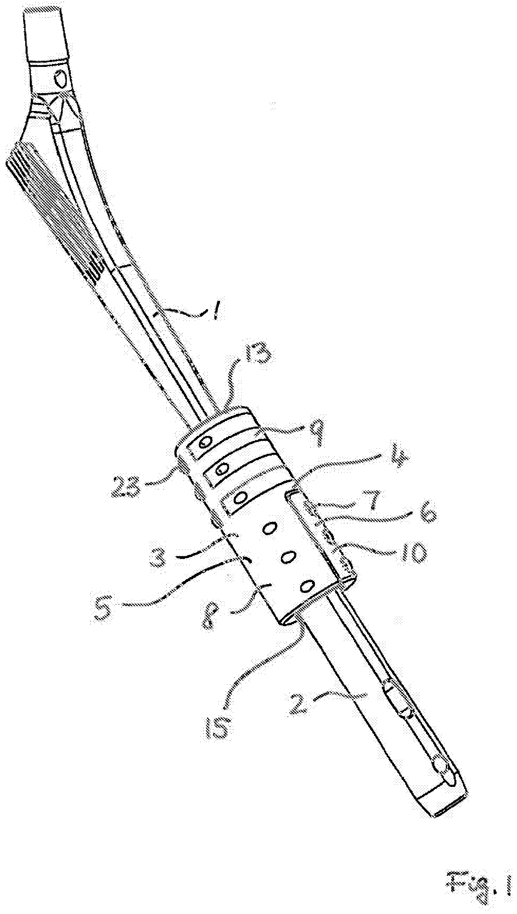

Im Folgenden wird zunächst eine im gezeigten Ausführungsbeispiel als Endoprothesenanordnung ausgeführte Implantatanordnung unter Bezugnahme auf die Fig. 1 bis 6 beschrieben.

Fig. 1 zeigt eine perspektivische Darstellung einer Implantatanordnung, bei der ein erstes Implantatteil 1 und ein zweites Implantatteil 2 endseitig mit Hilfe eines Zwischenkörpers 3 fest miteinander verbunden sind, wobei die beiden Implantatteile 1, 2 im gezeigten Ausführungsbeispiel als Endoprothesenteile gebildet sind. Das erste und das zweite Implantatteil 1, 2 sind für eine zumindest teilweise intramedulläre Anordnung vorgesehen. Nach dem Implantieren kann der Zwischenkörper 3 einen knöchernen Bereich im diaphysären und / oder metaphysären Bereich des Knochens ersetzen. Bei der dargestellten Ausführungsform ist das erste Implantatteil 1 einer Hüftgelenksprothese zugeordnet, wohingegen das zweite Implantatteil 2 ein Nagelimplantat ist. Diese Ausgestaltung ist jedoch lediglich beispielhaft. Grundsätzlich können mit dem Zwischenstück 3 beliebige Endabschnitte von Implantatteilen fest miteinander verbunden werden, insbesondere derart, dass der Zwischenkörper 3 der Implantatanordnung zum Ersatz eines knöchernen Bereiches im diaphysären und / oder metaphysären Bereiches des Knochens dient.

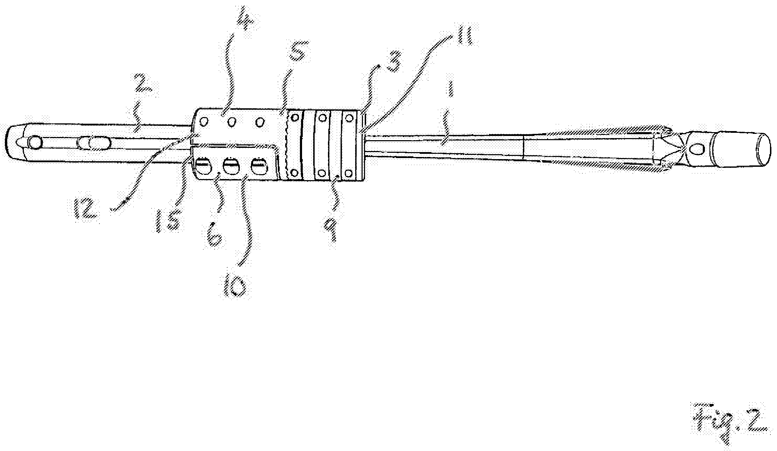

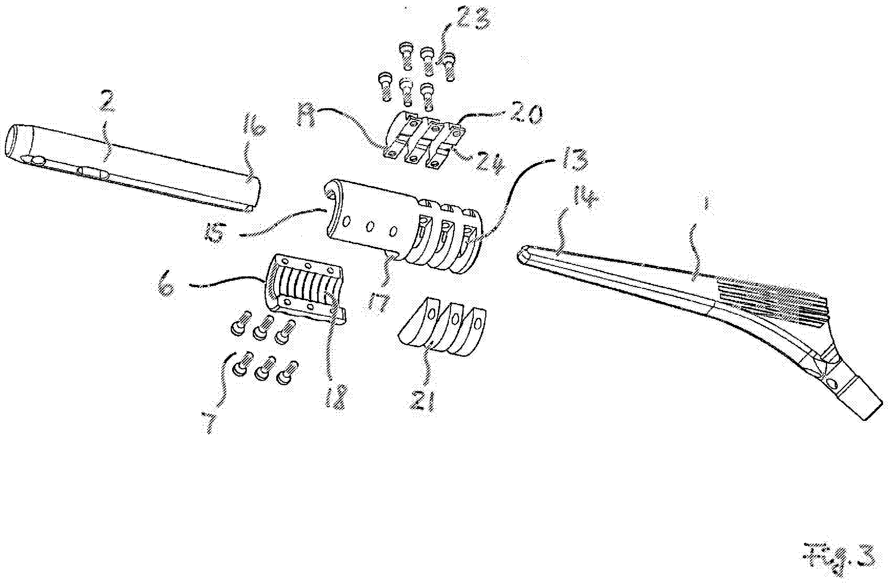

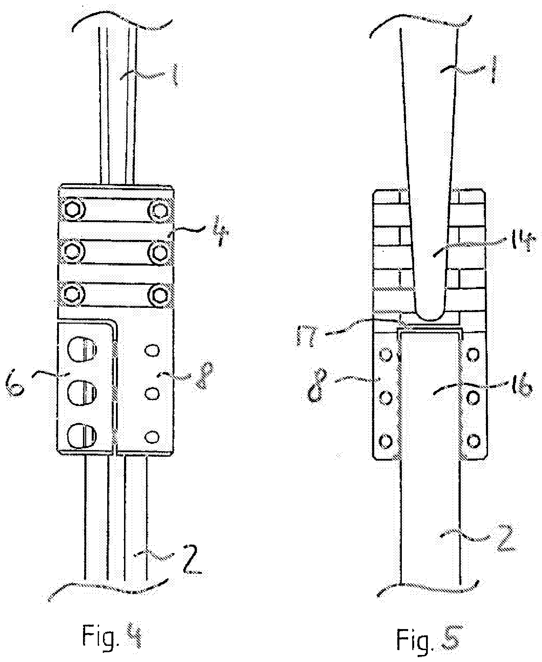

Die Fig. 2 und 3 zeigen weitere Darstellungen der Implantatanordnung aus Fig. 1. In den Fig. 4 und 5 ist ein Abschnitt mit dem Zwischenkörper 3 der Implantatanordnung aus Fig. 1 von vom und im Schnitt dargestellt.

Der Zwischenkörper 3 weist einen Basiskörper 4 auf. Der Basiskörper 4 ist in der dargestellten Ausführungsform mehrteilig gebildet, mit einem Basisteil 5 und einem Zusatzteil 6, welches mit Hilfe von Schrauben 7 lösbar am Basisteil 5 montiert wird. Gemäß Fig. 3 ist das Zusatzteil 6 in der gezeigten Ausführungsform als Halbschale gebildet, die auf einen zugeordneten Abschnitt 8 am Basisteil 5 aufsetzt, welcher ebenfalls als Halbschale gebildet ist, und mittels der Schrauben 7 hieran befestigt wird.

Der Basiskörper 4 verfügt über einen ersten Basiskörperabschnitt 9 und einen zweiten Basiskörperabschnitt 10, die im gezeigten Beispiel eine jeweilige Basiskörperhälfte bilden. Während der Basiskörper 4 im ersten Basiskörperabschnitt 9 mit einer einteiligen Wandung 11 ausgeführt ist, ist die Wandung 12 im zweiten Basiskörperabschnitt 10 mit Hilfe des Zusatzteils 6 und des zugeordneten Abschnitts 8 mehrteilig gebildet. Die Wandung 11 im ersten Basiskörperabschnitt 9 umgreift eine erste Aufnahme 13, die als Aufnahmetasche ausgeführt ist. Im zusammengebauten Zustand (vgl. Fig. 1 und 2) greift in die erste Aufnahmetasche 13 ein Endabschnitt 14 des ersten Implantatteils 1 ein. Vergleichbar ist im zweiten Basiskörperabschnitt 10 eine zweite Aufnahme 15 gebildet, in welcher ein zugeordneter Endabschnitt 16 des zweiten Implantatteils 2 eingreift. Auch die zweite Aufnahme 15 ist als Aufnahmetasche gebildet. Die beiden Aufnahmen 13, 15 sind durch eine Trennwand 17 (vgl. Fig. 5) voneinander getrennt. Die erste und die zweite Aufnahme 13, 15 werden von der jeweils zugeordneten Wandung 11, 12 umgriffen, die Teil des Basiskörpers 4 ist.

Der Endabschnitt 16 des zweiten Implantatteils 2 wird in der zweiten Aufnahme 15 fixiert mittels Klemmen, indem dass Zusatzteil 6 mit Hilfe der Schrauben 7 am zugeordneten Abschnitt 8 des Basiskörpers 4 angeschraubt wird. Zur verbesserten Fixierung sind in der zweiten Aufnahme 15 rutschhemmende Oberflächenstrukturen 18 vorgesehen. Bei der gezeigten Ausführungsform ist die zweite Aufnahme 15 mit einer zylindrischen Innenform versehen.

Im Unterschied zur Fixierung des Endabschnitts 16 des zweiten Implantatteils 2 mit Hilfe der die zweite Aufnahme 15 umgreifenden Wandungsabschnitte des Zusatzteils 6 und des zugeordneten Abschnitts 8 erfolgt das Fixieren des Endabschnitts 14 des ersten Implantatteils 1 in der ersten Aufnahme 13 mit Hilfe einer Klemmeinrichtung 19, die gemäß Fig. 3 mit Klemmbauteilen 20 und Gegenklemmbauteilen 21 gebildet ist. Die Klemmbauteile 20 sowie die Gegenklemmbauteile 21 sind nach der Montage in zugeordneten Klemmbauteilaufnahmen 22 angeordnet, die bei der gezeigten Ausführungsform einer regelmäßigen, sich in axialer Richtung erstreckenden Anordnung entsprechend am Basiskörper 4 hergestellt sind. In der gezeigten Ausführung sind die Klemmbauteile 20 und die Gegenklemmbauteile 21 als flache Bauteile ausgeführt, die seitlich im Wesentlichen formschlüssig in die zugeordneten Klemmbauteilaufnahmen 22 eingefügt sind. Das Zusammenhalten der Klemmbauteile 20 und der Gegenklemmbauteile 21 erfolgt mit Hilfe weiterer Schrauben 23. Mittels der weiteren Schrauben 23 werden die Klemmbauteile 20 sowie die Gegenklemmbauteile 21 dann auch gemeinsam und aufeinander zulaufend in eine jeweilige Klemmstellung gebracht, was mittels Relativverlagerung bezüglich der die erste Aufnahme 13 umgebenden Wandung 11 erfolgt, so dass schließlich der Endabschnitt 14 des ersten Implantatteils 1 in der ersten Aufnahme 13 geklemmt wird.

Bei der Ausführung nach Fig. 3 weisen die Klemmbauteile 20 sowie die Gegenklemmbauteile 21 auf der der ersten Aufnahme 13 zugewandten Seite eine oberflächenseitige Klemmstruktur 24 auf, die, wie sich aus Fig. 3 ergibt, zumindest für die Klemmbauteile 20 verschieden ist, derart, dass sich die Weite oder Breite der oberflächenseitigen Klemmstruktur 24 zur Mitte des Basiskörpers 4 hin verkleinert. Hierdurch ist eine konische oder sich verjüngende Passstruktur mit Hilfe der mehreren Gegenklemmbauteile 21 im Bereich der ersten Aufnahme 13 bereitgestellt. Die Klemmbauteile 20 können wahlweise über eine vergleichbare oder eine andere oberflächenseitige Klemmstruktur verfügen.

Alternativ kann auch vorgesehen sein (nicht dargestellt), dass am Basiskörper 4 lediglich die Klemmbauteile 20 angeordnet sind, wohingegen die Gegenklemmbauteile 21 nicht vorgesehen sind, derart, dass die die erste Aufnahme 13 umgebende Wandung 11 hier vollflächig und geschlossen ausgeführt ist, so dass beim Klemmen des Endabschnitts 14 in der ersten Aufnahme 13 dieser mittels der Klemmbauteile 20 gegen die Innenwand in der ersten Aufnahme 13 gedrückt wird.

Aus den Darstellungen in den Fig. 1 bis 5 ergibt sich, dass die Schraubrichtung und somit die Wirkrichtung der Schrauben 7 einerseits und der weiteren Schrauben 23 andererseits quer zueinander verlaufen.

Während im zweiten Basiskörperabschnitt 10 der Endabschnitt 16 bei gelöstem Zusatzbauteil 6 von oben auf den zugeordneten Abschnitt 8 aufgelegt werden kann, um anschließend die zweite Aufnahme 15 vervollständigend das Zusatzbauteil 6 hierauf aufzulegen, ist der Endabschnitt 14 des ersten Implantatteils 1 in die erste Aufnahme 13 durch die stirnseitige Eintrittsöffnung einzuschieben oder einzustecken.

Es kann vorgesehen sein (nicht dargestellt), auch im Bereich des zweiten Basiskörperabschnitts 10 Klemmbauteile vergleichbar dem Klemmbauteil 20 und den Gegenklemmbauteilen 21 in zugeordneten Aufnahmen vorzusehen.

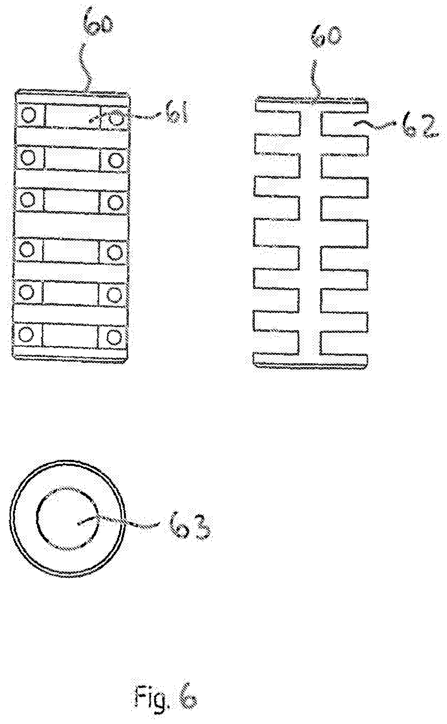

Fig. 6 zeigt einen weiteren Zwischenkörper 60, welcher, vergleichbar dem ersten Basiskörperabschnitt 9 bei der Ausführung in den Fig. 1 bis 5, mit Klemmbauteilaufnahmen 62 versehen ist, hier aber über die gesamte Länge des Basiskörpers 4. Die Anordnung der Klemmbauteilaufnahmen 62 mit zugeordneten Klemmbauteilen erstreckt sich hier über die gesamte axiale Länge des Zwischenkörpers 60.

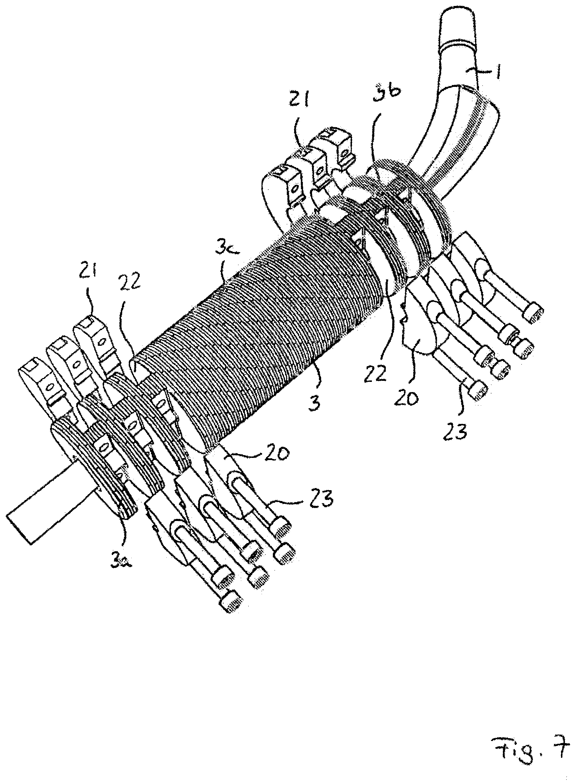

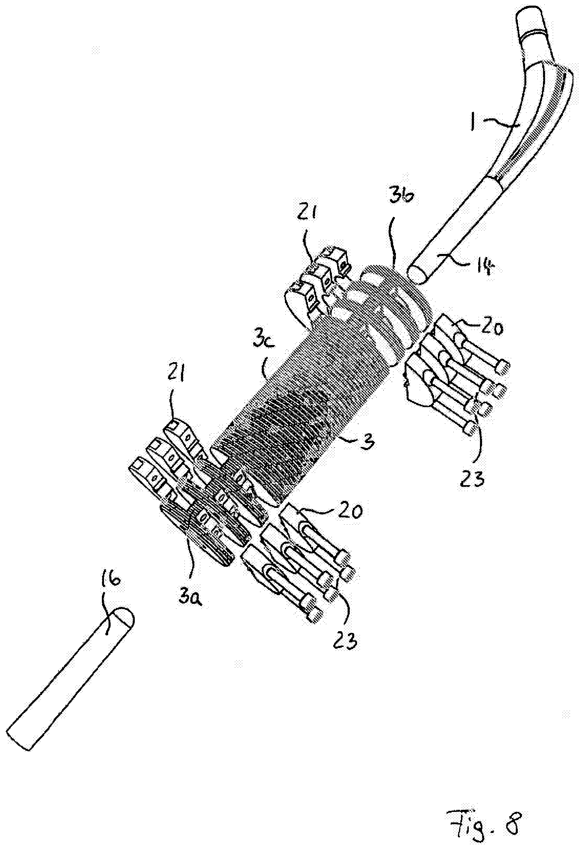

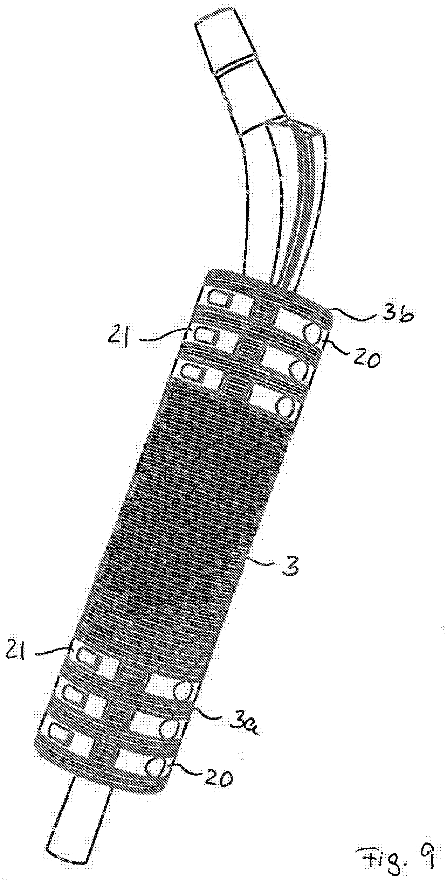

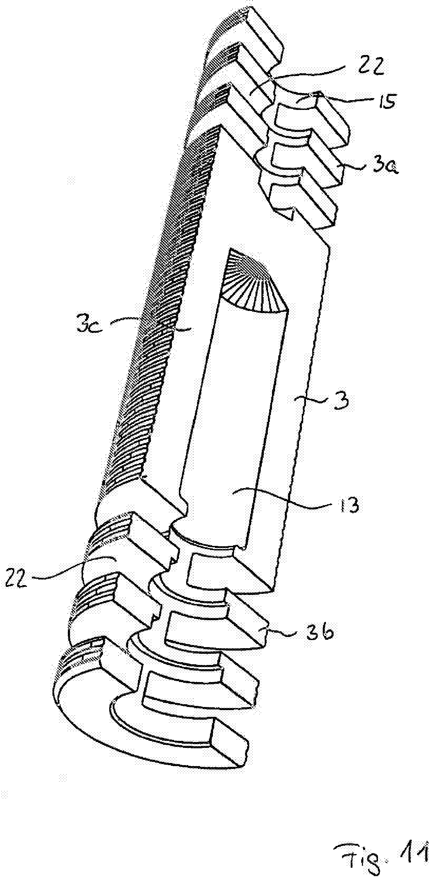

Nachfolgend wird unter Bezugnahme auf die Fig. 7 bis 11 eine weitere Implantatanordnung mit einem Zwischenkörper beschrieben. Für gleiche Merkmale werden dieselben Bezugszeichen wie in den vorangehenden Figuren verwendet.

Bei dem Zwischenkörper 3 der Implantatanordnung in den Fig. 7 bis 11 sind Endabschnitte 3a, 3b jeweils mit Klemmbauteilaufnahmen 22 gebildet, in die zum Klemmen der Endabschnitte 14, 16 Klemmbauteile 20 und Gegenklemmbauteile 21 eingeführt werden. In einem mittleren Bereich 3c ist der Zwischenkörper 3 frei von den Aufnahmen 22.

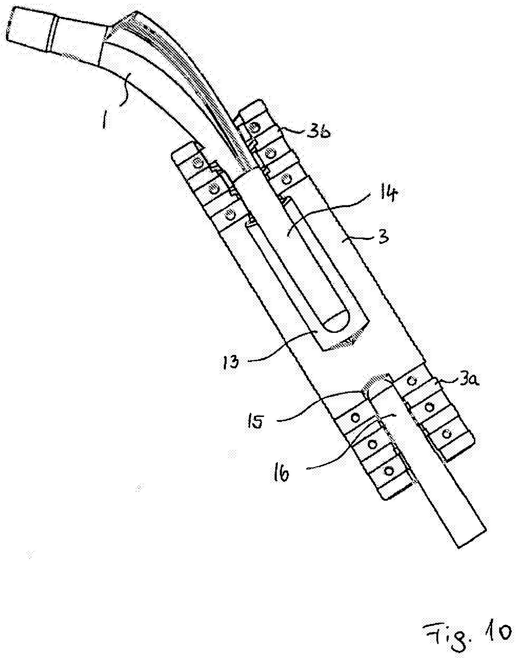

Wie sich insbesondere aus Fig. 10 ergibt, sind die erste und die zweite Aufnahme 13, 15 jeweils als ein Sackloch gebildet. Insbesondere Fig. 10 zeigt auch, dass je nach Anwendungsbeispiel der eingesteckte Endabschnitt 14 sich wahlweise bis zur Mitte hin oder sogar darüber hinaus des Implantatteils 1 erstrecken kann. Bei der weiteren Implantatanordnung verfügen die beiden Aufnahmen 13, 15 über eine unterschiedliche axiale Tiefe.

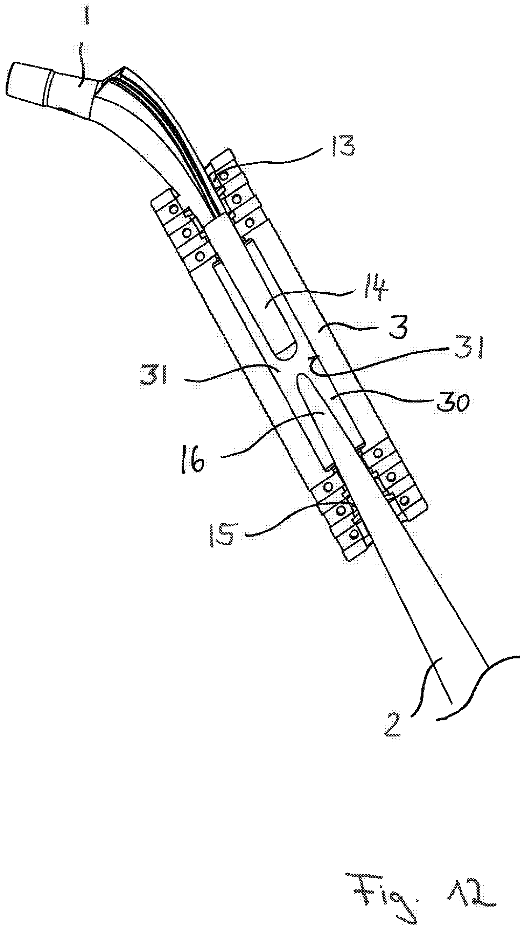

Fig. 12 zeigt eine schematische Darstellung einer Implantanordnung, wobei das Zwischenstück 3 im Schnitt dargestellt ist. Die Aufnahmen 13, 15 sind miteinander verbunden, so dass eine durchgehende Aufnahme 30 gebildet ist. Hierdurch ist es ermöglicht, beide Implantatteile 1, 2 mit flexibler Einstecktiefe in die durchgehende Aufnahme 30 einzuschieben. In einem mittleren Abschnitt 31 verfügt die durchgehende Aufnahme 30 über einen gleichbleibenden Durchmesser, derart, dass die Endabschnitte 14, 16 im mittleren Abschnitt 31 umlaufend freiliegend angeordnet sind, also ohne Berührungskontakt mit einer Innenwand 32 im mittleren Bereich 31.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Zwischenkörper (3; 60) für ein Knochenimplantat zum Ersatz eines knöchernen Bereiches, mit
- einem Basiskörper (4),
- einer Aufnahme (13), die sich im Basiskörper (4) in axialer Richtung erstreckend und hierbei von einer Wandung (11) des Basiskörpers (4) umgriffen gebildet und konfiguriert ist, einen Endabschnitt (14) eines intramedullären Implantatteils (1) umgreifend aufzunehmen, und
- einer der Aufnahme (13) zugeordneten Klemmeinrichtung, wobei die Klemmeinrichtung mit mehreren Klemmbauteilen (20) gebildet ist, die an der die Aufnahme (13) umgreifenden Wandung (11) des Basiskörpers (4) angeordnet sind und die unabhängig voneinander oder zum Teil gemeinsam zum Klemmen des zuvor in die Aufnahme (13) eingeführten Endabschnittes (14) relativ zur Wandung (11) des Basiskörpers (4), die dann die Aufnahme (13) und den eingeführten Endabschnitt (14) umgreift, in eine Klemmstellung verlagerbar sind, also zur Aufnahme (13) hin und hierbei den Endabschnitt (14) in der Aufnahme (13) klemmend,
**dadurch gekennzeichnet, dass** die mehreren Klemmbauteile (20) mittels Schrauben (7; 23) in die Klemmstellung verlagerbar sind und eine sich in axialer Richtung des Basiskörpers (4) erstreckende Anordnung von Klemmelementen bilden.

2. Zwischenkörper (3; 60) nach Anspruch 1, **dadurch gekennzeichnet, dass** die die Aufnahme (13) umgreifende Wandung (11) des Basiskörpers (4) einteilig ausgeführt ist.

3. Zwischenkörper (3; 60) nach Anspruch 1 oder 2, **dadurch gekennzeichnet, dass** die mehreren Klemmbauteile (20) zumindest in einer Klemmstellung wenigstens abschnittsweise in einer zugeordneten Klemmbauteilaufnahme (22) im Bereich der umgreifenden Wandung (11) des Basiskörpers (4) angeordnet sind.

4. Zwischenkörper (3; 60) nach mindestens einem der vorangehenden Ansprüche, **dadurch gekennzeichnet, dass** die mehreren Klemmbauteile (20) lösbar an der umgreifenden Wandung (11) aufgenommen sind.

5. Zwischenkörper (3; 60) nach mindestens einem der vorangehenden Ansprüche, **dadurch gekennzeichnet, dass** die mehreren Klemmbauteile (20) zum Klemmen des zuvor in die Aufnahme (13) eingeführten Endabschnittes (14) in die Aufnahme (13) hinein verlagerbar sind.

6. Zwischenkörper (3; 60) nach mindestens einem der vorangehenden Ansprüche, **dadurch gekennzeichnet, dass** für die mehreren Klemmbauteile (20) individuelle und voneinander verschiedene Klemmweiten in der Aufnahme (13) einstellbar sind.

7. Zwischenkörper (3; 60) nach mindestens einem der vorangehenden Ansprüche, **dadurch gekennzeichnet, dass** die sich in axialer Richtung des Basiskörpers (4) erstreckende Anordnung von Klemmelementen über die gesamte Länge des Basiskörpers gebildet ist.

8. Zwischenkörper (3; 60) nach mindestens einem der vorangehenden Ansprüche, **dadurch gekennzeichnet, dass** die mehreren Klemmbauteile (20) auf einer der Aufnahme (13) zugewandten Seite eine oberflächenseitige Klemmstruktur aufweisen.

9. Zwischenkörper (3; 60) nach mindestens einem der vorangehenden Ansprüche, **dadurch gekennzeichnet, dass** das auf einer Innenseite der die Aufnahme (13) umgreifenden Wandung (11) eine rutschhemmende Oberflächenprofilierung gebildet ist.

10. Zwischenkörper (3; 60) nach mindestens einem der vorangehenden Ansprüche, **dadurch gekennzeichnet, dass** die Klemmeinrichtung mit Spannelementen (7, 23) gebildet ist, mit denen quer zueinander ausgerichtete Klemmkräfte ausbildbar sind.

11. Zwischenkörper (3; 60) nach mindestens einem der vorangehenden Ansprüche, **dadurch gekennzeichnet, dass** die mehreren Klemmbauteile (20) mehrteilig ausgeführt sind.

12. Zwischenkörper (3; 60) nach mindestens einem der vorangehenden Ansprüche, **dadurch gekennzeichnet, dass** die mehreren Klemmbauteile (20) jeweils mit einem in die die Aufnahme (13) umgreifende Wandung (11) des Basiskörpers (4) eingeschraubten Schraubelement gebildet sind.

13. Implantatanordnung, mit einem Zwischenkörper (3; 60) nach mindestens einem der vorangehenden Ansprüche, bei der in die Aufnahme (13) im Basiskörper (4) ein Endabschnitt (14) eines intramedullären Implantatteils (1) wie Endoprothese oder Nagel eingeführt und hierin mittels Klemmung fixiert ist.

## Claims

1. An intermediate body (3; 60) for a bone implant for replacement of an osseous area, comprising
- a base body (4),
- a reception (13) which extends in axial direction in the base body (4) and is surrounded here by a wall (11) of the base body (4) and which is configured to receive in a surrounding manner an end portion (14) of an intramedullary implant part (1), and
- a clamping device, associated with the reception (13), wherein the clamping device is formed with a plurality of clamping components (20), which are arranged on the wall (11) of the base body (4) surrounding the reception (13), and which are movable into a clamping position independently of one another or partially jointly, for clamping the end portion (14), previously introduced into the reception (13), relative to the wall (11) of the base body (4), which then surrounds the reception (13) and the introduced end portion (14), therefore towards the reception (13) and clamping here the end portion (14) in the reception (13),
**characterized in that** the plurality of clamping components (20) are movable into the clamping position by means of screws (7; 23), and form an arrangement of clamping elements extending in axial direction of the base body (4).

2. The intermediate body (3; 60) according to Claim 1, **characterized in that** the wall (11) of the base body (4), surrounding the seat (13), is formed in one piece.

3. The intermediate body (3; 60) according to Claim 1 or 2, **characterized in that** the plurality of clamping components (20) are arranged at least in a clamping position at least partially in an associated clamping component seat (22) in the region of the surrounding wall (11) of the base body (4).

4. The intermediate body (3; 60) according to at least one of the preceding claims, **characterized in that** the plurality of clamping components (20) are held detachably on the surrounding wall (11).

5. The intermediate body (3; 60) according to at least one of the preceding claims, **characterized in that** the plurality of clamping components (20), for clamping the end portion (14) previously introduced into the reception (13), are movable into the reception (13).

6. The intermediate body (3; 60) according to at least one of the preceding claims, **characterized in that** individual clamping widths, differing from one another, are able to be set in the reception (13) for the plurality of clamping components (20).

7. The intermediate body (3; 60) according to at least one of the preceding claims, **characterized in that** the arrangement of clamping elements, extending in axial direction of the base body (4), is formed over the entire length of the base body.

8. The intermediate body (3; 60) according to at least one of the preceding claims, **characterized in that** the plurality of clamping components (20) have a surface-side clamping structure on a side facing the seat (13).

9. The intermediate body (3; 60) according to at least one of the preceding claims, **characterized in that** a slip-resistant surface profiling is formed on an inner side of the wall (11) surrounding the reception (13).

10. The intermediate body (3; 60) according to at least one of the preceding claims, **characterized in that** the clamping device is formed with tensioning elements (7, 23), by which clamping forces aligned transversely to one another can be formed.

11. The intermediate body (3; 60) according to at least one of the preceding claims, **characterized in that** the plurality of clamping components (20) are formed having several parts.

12. The intermediate body (3; 60) according to at least one of the preceding Claims, **characterized in that** the plurality of clamping components (20) are formed respectively with a screw element screwed into the wall (11) of the base body (4) surrounding the reception (13).

13. An implant arrangement, with an intermediate body (3; 60) according to at least one of the preceding claims, in which an end portion (14) of an intramedullary implant part (1) such as an endoprosthesis or pin is introduced into the reception (13) in the base body (4) and is fixed herein by means of clamping.

## Revendications

1. Corps intermédiaire (3 ; 60) pour un implant osseux destiné à remplacer une zone osseuse, comprenant
- un corps de base (4),
- une réception (13) qui est formée et configurée en s'étendant dans le sens axial dans le corps de base (4) et entourée ainsi par une paroi (11) du corps de base (4) pour recevoir un tronçon d'extrémité (14) d'une partie d'implant intramédullaire (1), et
- un dispositif de serrage correspondant à la réception (13), dans lequel le dispositif de serrage est formé de plusieurs pièces de serrage (20) qui sont disposées sur la paroi (11) du corps de base (4) entourant la réception (13) et qui peuvent être déplacées dans une position de serrage, indépendamment l'un de l'autre ou partiellement ensemble pour serrer le tronçon d'extrémité (14) introduit auparavant dans la réception (13) par rapport à la paroi (11) du corps de base (4), qui entoure alors la réception (13) et le tronçon d'extrémité (14) introduit, c'est à dire en direction de la réception (13) et en serrant ainsi le tronçon d'extrémité (14) dans la réception (13),
**caractérisé en ce que** la pluralité de pièces de serrage (20) peut être déplacée dans la position de serrage au moyen de vis (7 ; 23) et forme un agencement d'éléments de serrage s'étendant dans le sens axial du corps de base (4).

2. Corps intermédiaire (3 ; 60) selon la revendication 1, **caractérisé en ce que** la paroi (11) du corps de base (4) entourant la réception (13) est conçue d'un seul tenant.

3. Corps intermédiaire (3 ; 60) selon la revendication 1 ou 2, **caractérisé en ce que** la pluralité de pièces de serrage (20) est disposée au moins dans une positon de serrage au moins par tronçons dans une réception (22) de pièces de serrage au niveau de la paroi (11) d'entourage du corps de base (4).

4. Corps intermédiaire (3 ; 60) selon au moins l'une des revendications précédentes, **caractérisé en ce que** la pluralité de pièces de serrage (20) est reçue de façon amovible sur la paroi (11) d'entourage.

5. Corps intermédiaire (3 ; 60) selon au moins l'une des revendications précédentes, **caractérisé en ce que** la pluralité de pièces de serrage (20) est mobile vers l'intérieur dans la réception (13) pour serrer le tronçon d'extrémité (14) introduit auparavant dans la réception (13).

6. Corps intermédiaire (3 ; 60) selon au moins l'une des revendications précédentes, **caractérisé en ce que** des largeurs de serrage individuelles et différentes l'une de l'autre peuvent être réglées dans la réception (13) pour la pluralité de pièces de serrage (20).

7. Corps intermédiaire (3 ; 60) selon au moins l'une des revendications précédentes, **caractérisé en ce que** l'agencement d'éléments de serrage s'étendant dans le sens axial du corps de base (4) est formé sur toute la longueur du corps de base.

8. Corps intermédiaire (3 ; 60) selon au moins l'une des revendications précédentes, **caractérisé en ce que** la pluralité de pièces de serrage (20) présente une structure de serrage côté surface sur un côté tourné vers la réception (13).

9. Corps intermédiaire (3 ; 60) selon au moins l'une des revendications précédentes, **caractérisé en ce qu'**un profilé de surface anti-dérapant est formé sur un côté intérieur de la paroi (11) entourant la réception (13).

10. Corps intermédiaire (3 ; 60) selon au moins l'une des revendications précédentes, **caractérisé en ce que** le dispositif de serrage est formé d'éléments de tension (7, 23) avec lesquels des forces de serrage dirigées transversalement les unes par rapport aux autres, peuvent être appliquées.

11. Corps intermédiaire (3 ; 60) selon au moins l'une des revendications précédentes, **caractérisé en ce que** la pluralité de pièces de serrage (20) est conçue en plusieurs parties.

12. Corps intermédiaire (3 ; 60) selon au moins l'une des revendications précédentes, **caractérisé en ce que** la pluralité de pièces de serrage (20) est formée respectivement par un élément de vissage vissé dans la paroi (11) du corps de base (4) entourant la réception (13).

13. Corps intermédiaire (3 ; 60) selon au moins l'une des revendications précédentes, dans lequel un tronçon d'extrémité (14) d'une partie d'implant intramédullaire (1) comme une endoprothèse ou un clou, est introduit dans la réception (13) dans le corps de base (4) et y est fixé par serrage.
